# EUROPÄISCHE PATENTSCHRIFT

(11) **EP 2 041 035 B1**
(45) Veröffentlichungstag und Bekanntmachung des Hinweises auf die Patenterteilung: **28.04.2010**
(21) Anmeldenummer: 07786044.3
(22) Anmeldetag: 12.07.2007
(51) Int. Cl.: C02F 3/28, C02F 1/00

(54) **REAKTOR MIT ZULAUFVERTEILSYSTEM ZUR ANAEROBEN ABWASSERREINIGUNG**
REACTOR COMPRISING A SUPPLY DISTRIBUTION SYSTEM FOR ANAEROBIC WASTE WATER TREATMENT
RÉACTEUR DOTÉ D'UN SYSTÈME DE RÉPARTITION DE L'ALIMENTATION POUR L'ÉPURATION ANAÉROBIE D'EAUX USÉES

(30) Priorität: 13.07.2006 DE 102006032489; 08.09.2006 DE 202006013811 U
(43) Veröffentlichungstag der Anmeldung: 01.04.2009
(73) Patentinhaber: Meri Entsorgungstechnik für die Papierindustrie GmbH, 81673 München (DE); AQUATYX WASSERTECHNIK GMBH, D-88212 Ravensburg (DE)
(72) Erfinder: MENKE, Lukas, 81545 München (DE); TROUBOUNIS, Georgios, 80331 München (DE); KNÖRLE, Ulrich, 88289 Waldburg (DE); EFINGER, Dieter, 84036 Kumhausen (DE)
(74) Vertreter: Manitz, Finsterwald & Partner GbR
(86) Internationale Anmeldenummer: PCT/EP2007/006216
(87) Internationale Veröffentlichungsnummer: WO 2008/006596

(56) Entgegenhaltungen:
- EP-A- 0 153 299
- EP-A- 1 553 058
- DE-A1- 4 208 148
- US-A1- 2006 065 593

## Beschreibung

Die vorliegende Erfindung betrifft einen Reaktor zur anaeroben Reinigung von Abwasser, insbesondere von Abwasser aus der Papierindustrie, umfassend einen Reaktorbehälter, wenigstens eine im unteren Bereich des Reaktorbehälters angeordnete Zulaufleitung zur Zuführung von zu reinigendem Abwasser in den Reaktor, wenigstens einen Zulaufverteiler zur Vermischung des dem Reaktor zugeführten Abwassers mit dem in dem Reaktor befindlichen Medium, wenigstens einen am oberen Reaktorbehälter angeordneten Überlauf zum Abführen von gereinigtem Wasser sowie wenigstens einen Abscheider.

Zur Abwasserreinigung sind eine Vielzahl von mechanischen, chemischen sowie biologischen Verfahren und entsprechende Reaktoren bekannt. Bei der biologischen Abwasserreinigung wird das zu reinigende Abwasser mit aeroben oder anaeroben Mikroorganismen kontaktiert, welche die in dem Abwasser enthaltenen organischen Verunreinigungen im Falle von aeroben Mikroorganismen überwiegend zu Kohlendioxid, Biomasse und Wasser und im Falle von anaeroben Mikroorganismen vorwiegend zu Kohlendioxid und Methan und nur zu einem geringen Teil zu Biomasse abbauen. Dabei werden die biologischen Abwasserreinigungsverfahren in jüngster Zeit zunehmend mit anaeroben Mikroorganismen durchgeführt, weil bei der anaeroben Abwasserreinigung nicht unter hohem Energieaufwand Sauerstoff in den Bioreaktor eingeführt werden muss, bei der Reinigung energiereiches Biogas erzeugt wird, welches nachfolgend zur Energiegewinnung eingesetzt werden kann, und deutlich geringere Mengen an Überschussschlamm erzeugt werden. Je nach der Art und Form der eingesetzten Biomasse werden die Reaktoren für die anaerobe Abwasserreinigung in Kontaktschlammreaktoren, UASB-Reaktoren, EGSB-Reaktoren, Festbettreaktoren und Fließbettreaktoren unterteilt. Während die Mikroorganismen bei Festbettreaktoren an ortsfesten Trägermaterialien und die Mikroorganismen bei Fließbettreaktoren auf frei beweglichen, kleinem Trägermaterial anhaften, werden die Mikroorganismen bei den UASB- und EGSB-Reaktoren in Form von sogenannten Pellets eingesetzt. Im Unterschied zu UASB-(upflow anaerobic sludge blanket; anaerobe Aufströmschlammbett)-Reaktoren sind EGSB-(expanded granular sludge bed; expandierte, granuläre Schlammbett)-Reaktoren höher und weisen bei gleichem Volumen eine deutlich kleinere Grundfläche auf.

Bei den UASB- und EGSB-Reaktoren wird dem Reaktor über einen Zulauf im unteren Reaktorbereich kontinuierlich zu reinigendes Abwasser oder eine Mischung aus zu reinigendem Abwasser und bereits gereinigtem Abwasser aus dem Ablauf des Anaerobreaktors (im nachfolgenden "Reaktorzulauf' genannt) zugeführt und durch ein oberhalb des Zulaufs befindliches, Mikroorganismenpellets enthaltendes Schlammbett geführt. Beim Abbau der organischen Verbindungen aus dem Abwasser bilden die Mikroorganismen insbesondere Methan und Kohlendioxid enthaltendes Gas (welches auch als Biogas bezeichnet wird), das sich teilweise in Form kleiner Bläschen an den Mikroorganismenpellets anlagert und teilweise in Form freier Gasbläschen in dem Reaktor nach oben steigt. Aufgrund der angelagerten Gasbläschen sinkt das spezifische Gewicht der Pellets, weshalb die Pellets in dem Reaktor nach oben steigen. Um das gebildete Biogas und die aufsteigenden Pellets von dem Wasser zu trennen, sind in dem mittleren und/oder oberen Teil des Reaktors Abscheider zumeist in Form von Gashauben angeordnet, unter deren First sich Biogas ansammelt, welches ein Gaspolster ausbildet, worunter eine Flotationsschicht aus Mikroorganismenpellets und Abwasser befindlich ist. Von Gas und Mikroorganismenpellets befreites, gereinigtes Wasser steigt in dem Reaktor nach oben und wird am oberen Ende des Reaktors über Überläufe abgezogen. Derartige Verfahren und entsprechende Reaktoren sind beispielsweise in der EP 0 170 332 A1 und in der EP 1 071 636 B1 beschrieben.

Besonders wichtig bei den zuvor beschriebenen Verfahren ist die gleichmäßige Verteilung des dem Reaktor über den Zulauf zugeführten Abwassers über den Reaktorquerschnitt, um eine gute Vermischung der in dem Reaktor befindlichen Schlammpellets, des in dem Reaktor befindlichen Wassers und des zugefügten Abwassers zu erreichen. Um diese Erfordernisse zu erfüllen, wurde bereits eine Vielzahl von mit entsprechenden Zulaufverteilern ausgestatteten Reaktoren vorgeschlagen.

Aus der EP 0 539 430 B1 ist ein Bioreaktor mit einem Reaktorbehälter bekannt, welcher an dem unteren Ende des Reaktorbehälters ein wenigstens eine Zulaufleitung umfassendes Zulauf Einlasssystem aufweist, welches durch eine im Wesentlichen kegelförmig angeordnete Abtrennung von der die Mikroorganismenpellets enthaltenden Reaktionskammer getrennt ist, wobei sich die Abtrennung über den gesamten Reaktorquerschnitt erstreckt. Dabei sind die Ausströmöffnungen der Zulaufleitungen wenigstens teilweise tangential ausgerichtet und an der Abtrennung ist wenigstens ein radialer Schlitz vorgesehen, der durch zwei radiale Kantenstreifen gebildet wird, die einander in einem gewissen vertikalen Abstand überlappen, wobei der wenigstens eine radiale Schlitz eine Verbindung zwischen der Zulauf-Einlasskammer und der Reaktionskammer bildet. Durch die Orientierung der Ausströmöffnung(en) der Zulaufleitung(en) und des wenigstens einen radialen Schlitzes soll eine bezüglich der Reaktorlängsachse kreisförmige Strömung des Zulaufwassers erreicht werden und durch die Anordnung der radialen Schlitze soll insbesondere nach Abschalten des Reaktors ein Eindringen von Feststoffen in das Zulauf-Einlassystem verhindert werden. Allerdings weist dieser Reaktor und das damit betriebene Verfahren eine Vielzahl von Nachteilen auf.

Aufgrund der 100 % des Reaktorquerschnitts ausfüllenden Abtrennung zwischen dem Zulauf-Einlasssystem und der Reaktorkammer sammeln sich in dem zugeführten Abwasser enthaltende Feststoffe innerhalb des Zulauf-Einlasssystems an und gelangen nicht in die Reaktionskammer. In dem Zulauf-Einlasssystem, das heißt unterhalb der kegelförmigen Abtrennung, abgelagerter Feststoff ist jedoch nur schwer aus dem Reaktor zu entfernen. Zudem muss für eine Entfernung der Feststoffe aus dem Zulauf-Einlasssystem der Reaktor abgeschaltet werden. Zum anderen kristallisiert in der Reaktorkammer insbesondere bei zu reinigendem Abwasser mit hohem Kalkgehalt an den Pellets Kalk aus, so dass ein Teil der Mikroorganismenschlammpellets, an denen der Kalk kristallisiert, ein hohes spezifisches Gewicht aufweist, so dass diese Pellets in dem Reaktor sedimentieren und sich am unteren Ende der kegelförmigen Abtrennung im Bereich der Reaktorwand absetzen. Auch diese Sedimente lassen sich nur unter höchstem Aufwand aus dem Reaktor entfernen. Zudem verringert sich durch das Sediment an der Reaktorwand der effektive Durchmesser des Reaktors, so dass sich das effektive Reaktionsvolumen verringert. Ferner werden durch die Sedimente mit zunehmender Betriebszeit des Reaktors die radialen Schlitze blockiert bzw. verstopft, was in einem großen Druckanstieg in dem Verteilersystem resultiert. Dies kann dazu führen, dass der Wasserspiegel in dem Gasabscheider signifikant ansteigt, weswegen es zu einem Stau in dem Gasabscheider kommen kann, was zu der Notwendigkeit des Abschaltens des Zulaufs führen kann. Weil zudem Kohlendioxid in dem Gasseparator ausgeschieden wird, steigt in der Abwasserrückführleitung der pH-Wert signifikant an, was zu einer Präzipitation von Kalk in dem Zulaufbereich führt, so dass das Präzipitat nach Stilllegen des Reaktors unter hohem Kostenaufwand entfernt werden muss. Aus diesen Gründen ist der vorgenannte Reaktor zur Reinigung von hoch kalkhaltigem Abwasser, wie Abwasser aus der Papierindustrie, das gleichzeitig auch sedimentierbare Verunreinigungen in Form von Papierfüllstoffen und dergleichen enthalten kann, verbesserungsbedürftig.

Aus der US 2006/0065593 A1 ist ein Reaktor zur anaeroben Reinigung von Abwasser bekannt, welcher einen Reaktorbehälter, wenigstens eine Zulaufleitung, wenigstens einen Überlauf sowie einen Abscheider umfasst, wobei an der Austrittsöffnung der Zulaufleitung ein T-Stück angeordnet ist und wobei in dem unteren Bereich des Reaktors eine Verteilungsplatte vorgesehen ist.

In der EP 0 153 299 A1 wird ein Reaktor zur anaeroben Behandlung von organischen Substraten beschrieben, welcher eine Zulaufleitung, eine Pufferzone, einen Verteilungsraum und einen von dem Verteilungsraum durch eine Zwischendecke abgetrennten Reaktionsraum umfasst, wobei die Pufferzone als ein konzentrisch um die Längsachse des Reaktors angeordneter Hohlzylinder ausgebildet ist, an dessen unterem Ende Verteilungsflügel vorgesehen sind, und wobei die Zulaufleitung in den oberen Bereich der Pufferzone mündet.

Aufgabe der vorliegenden Erfindung ist es, einen Reaktor mit einem Zulaufverteiler bereitzustellen, mit dem die vorgenannten Nachteile vermieden werden. Insbesondere soll der Reaktor zur Reinigung von Abwasser mit bei der anaeroben Behandlung zum Ausfällen neigenden Inhaltsstoffen, wie beispielsweise Kalk, insbesondere von Abwasser aus der Papierindustrie, geeignet sein und eine gleichmäßige Verteilung des Zulaufs über den gesamten Reaktorquerschnitt und eine optimale Fluidisierung des Schlammbettes ermöglichten. Zudem soll eine Verstopfung im Zulaufbereich, insbesondere im Bereich des Zulaufverteilers, zuverlässig vermieden werden und eine Anreicherung von Feststoffen im Bereich des Zulaufverteilers, insbesondere die Bildung von Toträumen aufgrund von Feststoffsedimenten, zuverlässig verhindert werden.

Erfindungsgemäß wird diese Aufgabe gelöst durch einen Reaktor mit den Merkmalen des Schutzanspruchs 1 und insbesondere durch einen Reaktor zur anaeroben Reinigung von Abwasser, umfassend einen Reaktorbehälter, wenigstens eine im unteren Bereich des Reaktorbehälters angeordnete Zulaufleitung zur Zuführung von zu reinigendem Abwasser in den Reaktor, wenigstens einen Zulaufverteiler zur Vermischung des dem Reaktor zugeführten Abwassers oder von recyceltem Material mit dem in dem Reaktor befindlichen Medium, wenigstens einen am oberen Reaktorbehälter angeordneten Überlauf zum Abführen von gereinigtem Wasser sowie wenigstens einen Abscheider, wobei der Zulaufverteiler wenigstens ein Ablenkmittel umfasst, wobei das wenigstens eine Ablenkmittel derart ausgestaltet ist, dass aus der wenigstens einen Zulaufleitung austretendes Abwasser in eine, vom Reaktorquerschnitt aus gesehen, kreisförmige Strömung umgelenkt wird, und, wobei 1 bis 80 % des Reaktorquerschnitts von dem wenigstens einen Ablenkmittel bzw. dem Zulaufverteiler ausgefüllt werden.

Durch den wenigstens ein Ablenkmittel umfassenden Zulaufverteiler und die derartige Ausgestaltung des wenigstens einen Ablenkmittels, dass aus der Austrittsöffnung der wenigstens einen Zulaufleitung austretendes Abwasser in eine vom Reaktorquerschnitt aus gesehen, kreisförmige Strömung umgelenkt wird, wird eine hervorragende Durchmischung des dem Reaktor zugeführten Abwassers mit dem in dem Reaktor befindlichen Medium ermöglicht. Des Weiteren ermöglicht die durch das wenigstens eine Ablenkmittel bewirkte Strömungsführung des Zulaufabwassers eine gleichmäßige Verteilung des Zulaufwassers über den gesamten Reaktorquerschnitt. Indem das wenigstens eine Ablenkmittel bzw. der Zulaufverteiler weniger als 80 % des Reaktorquerschnitts ausfüllt, wird eine vollständige Unterteilung des Reaktors in zwei getrennte Zonen, nämlich ein Zulauf-Einlasssystem und einer darüber angeordneten Reaktionskammer, vermieden, so dass sich zum einen in dem Zulauf enthaltene Feststoffe nicht in dem Bereich des Zulaufverteilers ansammeln, sondern durch die aufwärts gerichtete Strömung in dem Reaktor nach oben strömen, und zum anderen aus dem oberen Reaktorbereich nach unten sinkende Sedimente nicht im Bereich des Zulaufverteilers sedimentieren, sondern gezielt in eine Zone geleitet werden, aus der sie simultan während des laufenden Prozesses aus dem Reaktor entnommen werden können. Insbesondere wird so die Ausbildung von Toträumen, welche den effektiven Reaktorquerschnitt verringern, vermieden. Insbesondere kann so auch ein Druckanstieg in dem Verteilersystem zuverlässig verhindert werden, so dass ein Anstieg des Wasserspiegels in dem Gasabscheider verhindert wird. Aufgrund dessen eignet sich der erfindungsgemäße Reaktor insbesondere zur anaeroben Reinigung von feststoffbeladenem Abwasser und insbesondere zur anaeroben Reinigung von Abwasser aus der Papierindustrie, welches sedimentierbare Verunreinigungen sowie einen hohen Gehalt an gelöstem Kalk aufweist.

Vorzugsweise füllt das wenigstens eine Ablenkmittel bzw. der Zulaufverteiler 2 bis 70 %, besonders bevorzugt 3 bis 60 % und ganz besonders bevorzugt 5 bis 50 % des Reaktorquerschnitts aus. Darunter wird im Sinne der vorliegenden Erfindung verstanden, dass das Integral des Anteils des/der Ablenkmittel(s) an jeder Querschnittsfläche des Reaktors 2 bis 70 % bzw. 3 bis 60 % bzw. 5 bis 50 % beträgt. Indem das wenigstens eine Ablenkmittel wenigstens 2 % bzw. 3 % bzw. 5 % des Reaktorquerschnitts ausfüllt, wird erreicht, dass der Reaktorzulauf in eine stabile, vom Reaktorquerschnitt aus gesehen kreisförmige Strömung umgelenkt wird, so dass eine exzellente Vermischung des Reaktorzulaufs mit dem im Reaktor befindlichen Medium erreicht wird. Durch die Ausfüllung von maximal 80 % bzw. 70 % bzw. 60 % bzw. 50 % des Reaktorquerschnitts durch das/die Ablenkmittel wird zudem erreicht, dass in dem zugeführten Abwasser enthaltene Feststoffe durch die kreisförmige, nach oben gerichtete Wasserströmung (Spiralströmung) in den Bereich der Schlammpelletzone des Reaktors aufsteigen und sich zum anderen aus dem oberen Reaktorbereich herabsinkender Feststoff mit hoher spezifischer Dichte nicht im Bereich des Zulaufverteilers ansammelt, sondern bis auf den Reaktorboden absinkt und von dort während des laufenden Prozesses aus dem Reaktor entnommen werden können.

In Weiterbildung des Erfindungsgedankens wird vorgeschlagen, die wenigstens eine Zulaufleitung außerhalb des Reaktorbehälters enden zu lassen und in der Wand des unteren Bereichs des Reaktorbehälters wenigstens einen Schlitz vorzusehen, durch den das aus der wenigstens einen Zulaufleitung austretende Abwasser in den unteren Bereich des Reaktorbehälters eintritt. Diese Ausführungsform ist zum einen konstruktiv einfach, weil auf Einrichtungen für den Durchtritt der Zulaufleitung durch den Reaktorbehälter verzichtet werden kann und ermöglicht es zum anderen, die Ablenkmittel unmittelbar im Bereich des Reaktorbehälters anzuordnen. Indem der wenigstens eine Schlitz in der Reaktorwand vorgesehen ist, kann auf eine Trennwand in dem Reaktor, insbesondere auf eine den gesamten Reaktorquerschnitt ausfüllende Trennwand in dem Reaktor mit den vorgenannten Nachteilen, verzichtet werden.

Gemäß einer bevorzugten Ausführungsform der vorliegenden Erfindung ist im Inneren des Reaktors im Bereich des wenigstens einen Schlitzes mindestens ein Ablenkmittel vorgesehen, welches derart ausgestaltet ist, dass das aus dem wenigstens einen Schlitz in den Reaktor eintretende Abwasser in eine, vom Reaktorquerschnitt aus gesehen, kreisförmige Strömung umgelenkt wird. Diese Ausführungsform, ermöglicht bei konstruktiv einfacher Ausgestaltung des Zulaufverteilers eine effektive Umlenkung des dem Reaktor zugeführten Abwassers in eine vom Reaktorquerschnitt aus gesehen kreisförmige Strömung.

Um eine besonders effektive Umlenkung des dem Reaktor zugeführten Abwassers in eine kreisförmige Strömung zu erreichen wird in Weiterbildung des Erfindungsgedankens vorgeschlagen, das wenigstens eine Ablenkmittel derart anzuordnen, dass dieses den wenigstens einen Schlitz zumindest teilweise überdeckt bzw. überlappt.

Zum Erreichen des vorgenannten Zwecks ist es insbesondere bevorzugt, wenn das wenigstens eine, den wenigstens einen Schlitz zumindest teilweise überdeckende bzw. überlappende Ablenkmittel im Wesentlichen die gleiche Grundform wie der wenigstens eine Schlitz aufweist.

Grundsätzlich können sowohl der wenigstens eine Schlitz als auch das wenigstens eine Ablenkmittel jede beliebige Geometrie aufweisen, wobei lediglich beispielsweise polygonale, kreisrunde, elliptische, ovale, quadratische und rechteckige Grundformen genannt seien. Vorzugsweise weisen der wenigstens eine Schlitz und das wenigstens eine Ablenkmittel die gleiche Grundform auf, wobei besonders gute Ergebnisse erzielt werden, wenn die Grundform sowohl des wenigstens einen Schlitzes als auch des wenigstens einen Ablenkmittels zumindest im Wesentlichen rechteckig ist.

Um auf gesonderte Befestigungseinrichtungen für das wenigstens eine Ablenkmittel verzichten zu können, ist das wenigstens eine Ablenkmittel bevorzugt mit einer seiner Seiten mit einer Seite des wenigstens einen Schlitzes verbunden. Dies lässt sich konstruktiv besonders einfach realisieren, wenn sowohl der wenigstens eine Schlitz als auch das wenigstens eine Ablenkmittel eine im Wesentlichen rechteckige Grundform aufweisen und das wenigstens eine Ablenkmittel mit seiner Längs- oder seiner Breitseite mit der Längs- oder der Breitseite des wenigstens einen Schlitzes verbunden ist.

Um beim Betrieb des Reaktors eine effektive Umlenkung des dem Reaktor durch den wenigstens einen Schlitz zugeführten Reaktorzulaufs in ein kreisförmige Strömung zu erreichen und andererseits beim Abschalten des Reaktors den wenigstens einen Schlitz einfach verschließen zu können, ist es gemäß einer weiteren bevorzugten Ausführungsform der vorliegenden Erfindung bevorzugt, dass das wenigstens eine Ablenkmittel mit dem wenigstens einen Schlitz, bspw. über ein Scharnier, drehbar verbunden ist.

Eine effektive Umlenkung des dem Reaktorinneren durch den wenigstens einen Schlitz zugeführten Abwassers wird insbesondere erreicht, wenn sich das wenigstens eine Ablenkmittel von der Verbindungsstelle des wenigstens einen Ablenkmittels mit dem wenigstens einen Schlitz aus gesehen relativ zu dem wenigstens einen Schlitz in einem Winkel zwischen 5° und 85°, bevorzugt zwischen 10° und 70°, besonders bevorzugt zwischen 20° und 50° und ganz besonders bevorzugt zwischen 30° und 40° erstreckt.

Um eine Absetzung von in dem Reaktormedium befindlichen Feststoffen an rechten Winkeln des Reaktorbehälters zu vermeiden, ist es bevorzugt, den unteren Teil des Reaktorbehälters nach unten konisch verjüngt auszubilden. Dadurch wird erreicht, dass an der Reaktorbehälterwand sedimentierender Feststoff bis zu dem tiefsten Punkt des Reaktorbodens absinkt und sich dort sammelt. In diesem Fall hat es sich als vorteilhaft erwiesen, den wenigstens einen Schlitz in dem sich konisch verjüngenden Teil des Reaktorbehälters und das wenigstens eine Ablenkmittel im Bereich des wenigstens einen Schlitzes vorzusehen.

Alternativ dazu ist es möglich und auch bevorzugt, den unteren Teil des Reaktorbehälters doppelkonisch auszubilden, wobei der obere Teil des Doppelkonus bezogen auf die Horizontalebene vorzugsweise einen kleineren Winkel als der untere Teil des Doppelkonus aufweist und der wenigstens eine Schlitz bevorzugt in dem oberen Teil des Doppelkonus vorgesehen ist. Bei dieser Ausführungsform beträgt der Winkel des oberen Teils des Doppelkonus, bezogen auf die Horizontalebene, vorzugsweise zwischen 20° und 50° und besonders bevorzugt zwischen 25° und 35° und Winkel des unteren Teils des Doppelkonus, ebenfalls bezogen auf die Horizontalebene, bevorzugt zwischen 30° und 70° und besonders bevorzugt zwischen 40° und 50°.

Bei der vorgenannten Ausführungsform ist der wenigstens eine Schlitz vorzugsweise in dem oberen Teil des Doppelkonus des Reaktorbehälters und das wenigstens eine Ablenkmittel im Bereich des wenigstens einen Schlitzes vorgesehen. Dadurch wird erreicht, dass an der Reaktorbehälterwand sedimentierender Feststoff bis zu dem tiefsten Punkt des Reaktorbodens im unteren Teil des Doppelkonus absinkt und sich dort sammelt und somit eine Verstopfung der im oberen Teil des Doppelkonus vorgesehenen wenigstens einen Schlitzes zuverlässig verhindert werden kann.

Um eine stabile Kreislaufführung des Reaktorzulaufs zu erreichen, hat es sich zudem als vorteilhaft erwiesen, in dem unteren Bereich des Reaktorbehälters wenigstens zwei Schlitze vorzusehen. Indem in dem Zulaufverteiler mehrere Ablenkmittel vorgesehen sind, ist es möglich, die einzelnen Ablenkmittel gleichmäßig über den Querschnitt des Reaktors zu verteilen, so dass in dem Zulaufverteiler, bezogen auf den Reaktorquerschnitt, mehrere ablenkmittelfreie Bereiche vorhanden sind und die Querschnittsfläche der einzelnen Ablenkmittel vergleichsweise gering ist. Die Anzahl und die Form der Ablenkmittel in dem Reaktor hängen unter anderem von dem Querschnitt des Reaktors und von der Menge des Reaktorzulaufs pro Zeiteinheit ab. Bei den für Reaktoren zur anaeroben Reinigung von Abwasser üblichen Dimensionen beträgt die Anzahl der in dem unteren Bereich des Reaktorbehälters vorgesehen Schlitze bevorzugt zwischen 5 und 200, besonders bevorzugt zwischen 25 und 150 und ganz besonders bevorzugt zwischen 50 und 75.

In Weiterbildung des Erfindungsgedankens wird vorgeschlagen, die einzelnen Schlitze von der Reaktorlängsachse aus gesehen in radialer Richtung voneinander beabstandet und untereinander durch die Reaktorbehälterwand getrennt in Reihe anzuordnen und in dem unteren Bereich des Reaktorbehälters 2 bis 50, bevorzugt 5 bis 30 und besonders bevorzugt 5 bis 15 solcher Reihen vorzusehen. Dies ermöglicht einerseits die Zusammenfassung der Abwasserzufuhr zu den einzelnen Schlitzen einer Reihe in Form einer Sammelleitung und andererseits die voneinander unabhängige Steuerung der Abwasserzufuhr zu den Schlitzen verschiedener Reihen.

Vorzugsweise sind die einzelnen Schlitze und/oder das wenigstens eine Ablenkmittel, von der Reaktorlängsachse aus radial nach außen gesehen, flächenproportional verteilt. Dies kann beispielsweise dadurch erreicht werden, dass, von der Reaktorlängsachse aus in radialer Richtung gesehen, auf jeder konzentrisch um die Reaktorlängsachse gedachten Kreisfläche gleich viele Schlitze bzw. Ablenkmittel vorgesehen sind, die einzelnen Schlitze bzw. Ablenkmittel jedoch mit zunehmender radialer Richtung größer dimensioniert sind. Ferner kann dies besonders bevorzugt dadurch erreicht werden, dass die einzelnen Schlitze bzw. die einzelnen Ablenkmittel jeweils die gleiche Größe aufweisen, aber von der Reaktorlängsachse aus in radialer Richtung gesehen, mit zunehmenden Abstand von der Reaktorlängsachse mehr Schlitze bzw. Ablenkmittel pro konzentrisch um die Reaktorlängsachse gedachter Kreisfläche angeordnet sind.

Aus diesem Grund ist es bevorzugt, zur Zuführung von zu reinigendem Abwasser über die Schlitze in den Reaktor unterhalb jeder Reihe von Schlitzen jeweils eine Sammelleitung, welche mit jeweils einer Zulaufleitung verbunden ist, vorzusehen. Dabei können die Sammelleitungen jede beliebige geometrische Form aufweisen, insbesondere ein, im Querschnitt gesehen, U-Profil, V-Profil oder rechteckiges Profil aufweisen. Alternativ dazu können die Sammelleitungen auch in der Form eines Halbrohres vorgesehen sein. Bevorzugt können die Sammelleitungen mit nicht konstantem Querschnitt ausgeführt sein. Besonders bevorzugt ist die Querschnittsänderung so gewählt, dass die Austrittsgeschwindigkeit des Reaktorzulaufes bei einer Ausführung der Sammelleitung mit mindestens zwei Schlitzen durch den Schlitz in den Reaktionsraum jeweils dieselbe Geschwindigkeit aufweist.

Um eine effektive Umlenkung des durch die Schlitze zugeführten Reaktorzulaufs zu erreichen, werden die Anzahl der einzelnen Ablenkmittel und die Dimensionen der einzelnen Ablenkmittel vorzugsweise so ausgewählt, dass 3 bis 50 %, bevorzugt 5 bis 40 % und besonders bevorzugt 10 bis 20 % des Reaktorquerschnitts von dem/den Ablenkmittel(n) ausgefüllt wird/werden.

Gemäß einer weiteren bevorzugten Ausführungsform der vorliegenden Erfindung ist vorgesehen, zusätzlich zu den zuvor beschriebenen Schlitzen und Ablenkmittel(n) oder alternativ zu den zuvor beschriebenen Schlitzen und Ablenkmittel(n) in dem Reaktor wenigstens ein als planare, mehrarmige Spirale ausgestaltetes Ablenkmittel vorzusehen. Durch eine planare, mehrarmige spiralförmige Ausgestaltung des wenigstens einen Ablenkmittels lässt sich eine Ablenkung des dem Reaktor zugeführten Abwassers in eine kreisförmige Strömung besonders einfach und zuverlässig erreichen.

Unter planarer, mehrarmiger Spirale wird im Sinne der vorliegenden Erfindung jede planare Spirale verstanden, welche wenigstens zwei Spiralarme umfasst, und zwar unabhängig von der konkreten Form der Spiralarme und unabhängig von deren Länge. Insbesondere schließt dieser Begriff auch Spiralen ein, deren einzelne Spiralarme das Zentrum der Spirale in einem Winkel von weniger als 360° oder weniger als 180° umgeben.

Besonders gute Ergebnisse werden insbesondere erhalten, wenn das wenigstens eine spiralförmige Ablenkmittel wenigstens vier und besonders bevorzugt zwischen vier und acht Spiralarme aufweist. Dadurch wird eine effektive Umlenkung des dem Reaktor zugeführten Abwassers in eine kreisförmige Strömung erreicht.

Grundsätzlich ist die vorliegende Erfindung nicht auf die konkrete Form der einzelnen Spiralarme des Ablenkmittels beschränkt, wobei insbesondere mit spiralförmigen Ablenkmitteln gute Ergebnisse erzielt werden, deren einzelne Spiralarme im Wesentlichen die Form einer Halbellipse aufweisen. Bei der letztgenannten Variante verläuft die Krümmung einzelner oder aller Spiralarme vorzugsweise in dieselbe Richtung.

Um eine besonders effektive Umlenkung des dem Reaktor zugeführten Abwassers zu erreichen, wird in Weiterbildung des Erfindungsgedankens vorgeschlagen, das wenigstens eine spiralförmige Ablenkmittel so auszugestalten, dass dieses vier bis acht im Wesentlichen halbelliptisch oder halbkreisförmig ausgebildete Spiralarme umfasst, von denen vorzugsweise zwei bis vier Spiralarme eine andere Länge aufweisen als der Rest der Spiralarme. Dadurch wird erreicht, dass das Abwasser dem Reaktor, über den Querschnitt des Reaktors gesehen, gleichmäßig zugeführt wird. Ein Beispiel für eine besonders geeignete Ausgestaltung dieser Ausführungsform der vorgenannten Erfindung ist ein Ablenkmittel mit acht im Wesentlichen halbelliptisch oder halbkreisförmig ausgebildeten Spiralarmen, von denen vier Spiralarme eine andere Länge aufweisen als die übrigen vier Spiralarme.

Um den konstruktiven Aufwand gering zu halten und eine gleichmäßige Wasserdurchfuhr durch das spiralförmige Ablenkmittel zu gewährleisten, wird in Weiterbildung des Erfindungsgedankens vorgeschlagen, in dem Zentrum des spiralförmigen Ablenkmittels eine Verteileinrichtung vorzusehen, wobei die Zulaufleitung in dem Zentrum des einen Ablenkmittels in die Verteileinrichtung mündet und das durch die Zulaufleitung geführte Abwasser über die Verteileinrichtung auf die einzelnen Spiralarme des Ablenkmittels verteilt wird.

Vorzugsweise weisen die Spiralarme des Ablenkmittels einen rohrförmigen Querschnitt auf. Bei dieser Ausführungsform der vorliegenden Erfindung sind die einzelnen Spiralarme des Ablenkmittels demnach, über den Radialschnitt gesehen, vollständig ummantelt, so dass ein Eindringen von Feststoffen in die Spiralarme zuverlässig verhindert werden kann, da die einzige Öffnung der Spiralarme, d.h. die Austrittsöffnung der Spiralarme, aufgrund der dort auströmenden Flüssigkeit für Feststoffe aus dem Reaktorinneren nicht zugänglich ist.

Gemäß einer weiteren bevorzugten, alternativen Ausführungsform der vorliegenden Erfindung wird vorgeschlagen, die Spiralarme des Ablenkmittels innen hohl und nach unten offen auszugestalten. In diesem Fall wird durch die Umfangsflächen der innen hohlen und nach unten offenen Spiralarme eine wirksame Umlenkung der durch das Ablenkmittel geführten Flüssigkeit erreicht. Bei dieser Ausführungsform ist/sind die Austrittsöffnung(en) der den einzelnen Spiralarmen des Ablenkmittels zugeordneten Zulaufleitung(en) vorzugsweise in den inneren Hohlraum der Spiralarme und besonders bevorzugt tangential zu einem gedachten, konzentrisch um die Reaktorlängsachse verlaufenden Kreis orientiert, so dass das aus der/den Austrittsöffnung(en) der Zulaufleitung(en) austretende Wasser auf die innere Umfangsfläche der Spiralarme trifft und dadurch entsprechend umgelenkt wird. Dabei wird eine kreisförmige Umlenkung des dem Reaktor zugeführten Wassers insbesondere dann zuverlässig erreicht, wenn das spiralförmige Ablenkmittel senkrecht zu der Reaktorlängsachse und konzentrisch um die Reaktorlängsachse herum angeordnet ist.

Bei der vorgenannten Ausführungsform sind die Spiralarme des Ablenkmittels vorzugsweise im Radialschnitt umgekehrt v-förmig, umgekehrt u-förmig, rechteckig, trapezförmig oder polygonal ausgestaltet. Insbesondere durch die umgekehrt v-förmige und umgekehrt u-förmige Ausgestaltung der Spiralarme im Radialschnitt wird erreicht, dass sich von oben auf das Ablenkmittel herabfallende Feststoffe nicht auf dem Umfang der Spiralarme ansammeln können, sondern über die Umfangsfläche der Spiralarme abrutschen und in dem Reaktor weiter nach unten absinken, bis diese den tiefsten Punkt des Reaktors erreichen.

Alternativ dazu können die Spiralarme des wenigstens einen spiralförmigen Ablenkmittels im Radialschnitt auch kreisförmig ausgestaltet sein, wobei der untere Teil des kreisförmigen Radialschnitts offen ist und an den beiden Öffnungsenden im Radialschnitt jeweils eine von den Spiralarmen weg weisende Schräge angebracht ist, mithin die Spiralarme des wenigstens einen spiralförmigen Ablenkmittels einen im Wesentlichen schlüssellochartigen Radialschnitt aufweist. Dadurch wird zum einen erreicht, dass von oben auf das Ablenkmittel herabsinkende Feststoffe von der Umfangsfläche der Spiralarme herabfallen und weiter nach unten in dem Reaktor sinken und andererseits verhindert, dass Feststoffe durch eine Seitenströmung von unten in den Hohlraum der Spiralarme eindringen und sich dort ablagern können.

Eine andere Alternative ist es, die Spiralarme des wenigstens einen spiralförmigen Ablenkmittels im Radialschnitt rautenförmig auszugestalten, wobei die unteren Teile der rautenförmigen Radialschnitte offen sind und an einem der beiden Öffnungsenden oder an beiden Öffnungsenden der einzelnen Radialschnitte der Spiralarme eine Schräge angebracht ist, welche vorzugsweise von den Spiralarmen nach außen führend angeordnet ist. Auch hierdurch wird zuverlässig vermieden, dass sich im Reaktor von oben auf das Ablenkmittel herabfallende Feststoffe auf dem Ablenkmittel ansammeln und dass Feststoffe von unten in den Hohlraum der Spiralarme des Ablenkmittels eindringen können.

Alternativ dazu können die Spiralarme des wenigstens einen spiralförmigen Ablenkmittels im Radialschnitt auch jede andere bekannte geometrische Form aufweisen, solange durch die Ausgestaltung der Spiralarme sichergestellt wird, dass keine Feststoffe in den Hohlraum der Spiralarme eintreten oder sich Feststoffe oben auf den Umfangsflächen der Spiralarme ansammeln können.

Um einerseits eine effektive Umlenkung des dem Reaktor zugeführten Abwassers zu erreichen und andererseits eine Sedimentation von in dem Reaktor herabsinkenden Feststoffen auf dem Ablenkmittel zu vermeiden, wird in Weiterbildung des Erfindungsgedankens vorgeschlagen, dass 5 bis 70 %, bevorzugt 10 bis 60 % und besonders bevorzugt 20 bis 50 % des Reaktorquerschnitts von dem wenigstens einen spiralförmigen Ablenkmittel ausgefüllt werden.

Vorzugsweise ist das wenigstens eine spiralförmige Ablenkmittel senkrecht zu der Reaktorlängsachse und konzentrisch um die Reaktorlänesachse herum angeordnet. Aufgrund dieser symmetrischen Anordnung wird die Umlenkung in eine kreisförmige Strömung begünstigt.

Gemäß einer weiteren bevorzugten Ausführungsform der vorliegenden Erfindung hat es sich als vorteilhaft erwiesen, den unteren Teil des Reaktors in der Form eines sich nach unten verjüngenden Einfachkonus oder Doppelkonus vorzusehen. Dadurch wird erreicht, dass aus dem oberen Reaktorteil herabsinkende Feststoffe mit hohem spezifischem Gewicht bis an die Spitze des Konus herabsinken und von dort abgeführt werden können. Hierdurch kann eine Ansammlung von Sedimenten im Bereich des Zulaufverteilers, welcher zu der Bildung von Toträumen und zu einer Reduzierung des effektiven Reaktorquerschnitts führt, zuverlässig vermieden werden. Dieser Effekt wird durch die durch den Zulaufverteiler erzeugte Spiralströmung maßgeblich verstärkt.

Aus dem vorgenannten Grund ist es zudem bevorzugt, an dem unteren Ende des Konus eine Abfuhrleitung anzuordnen.

In Weiterbildung des Erfindungsgedankens wird vorgeschlagen, im unteren Bereich des Konus eine Zulaufleitung vorzusehen, über die Abwasser, Frischwasser oder dergleichen in den unteren, konusförmigen Reaktorbereich eingeführt werden kann. Dadurch kann bei Bedarf eine Spülung des Reaktorbodens erfolgen. Dies ist beispielsweise dann sinnvoll, wenn Sediment aus dem unteren Reaktorbereich abgezogen wurde, um etwaige an den unteren Reaktorwänden anhaftende Sedimentreste aufzuwirbeln, so dass diese an dem untersten Punkt des Konus absinken und über die Abfuhrleitung aus dem Reaktor abgezogen werden können.

Um die Mikroorganismenpellets und das in dem Reaktor enthaltene Wasser im Kreislauf führen zu können, weist der Reaktor vorzugsweise wenigstens eine Sinkleitung, besonders bevorzugt eine konzentrisch um die Reaktorlängsachse angeordnete Sinkleitung, oder, sofern der Reaktor zwei Gasabscheider aufweist, zwei Sinkleitungen, auf, wobei das obere Ende der wenigstens einen Sinkleitung mit dem bzw. den Gasabscheider(n) verbunden ist/ sind und über die untere Austrittsöffnung der Sinkleitung(en) in dem/den Gasabscheider(n) abgetrennte Mikroorganismenpellets und Wasser in den unteren Reaktorbereich zurückgeführt werden können. Bei dieser Ausführungsform ist die untere Austrittsöffnung der wenigstens einen Sinkleitung oberhalb oder im Bereich der oberen Begrenzung des wenigstens einen Ablenkmittels angeordnet.

Sofern in dem Reaktor eine oder mehrere Sinkleitungen vorgesehen sind, sind deren untere Austrittsöffnungen vorzugsweise tangential zu einem gedachten, konzentrisch um die Längsachse angeordneten Kreis angeordnet. Bei zwei oder mehr vorgesehen Sinkleitungen können diese Austrittsöffnungen alle in derselben Richtung tangential zu einem gedachten, konzentrisch um die Längsachse angeordneten Kreis angeordnet sein oder alternativ dazu abwechselnd gegenläufig tangential zu einem gedachten, konzentrisch um die Längsachse angeordneten Kreis angeordnet sein.

Allerdings ist die vorliegende Erfindung nicht auf Reaktoren beschränkt, welche eine oder mehrere Sinkleitungen aufweisen. Vielmehr kann der erfindungsgemäße Reaktor auch ohne Sinkleitung betrieben werden. Zudem kann die wenigstens eine Sinkleitung, bezogen auf den Reaktorquerschnitt, auch an jeder beliebigen Stelle angeordnet sein. Schließlich ist es auch möglich, die wenigstens eine Sinkleitung außerhalb des Reaktorbehälters vorzusehen. Beispielsweise kann die wenigstens eine Sinkleitung außerhalb des Reaktorbehälters angeordnet sein. Darüber hinaus kann wenigstens eine Zufuhrleitung in die Sinkleitung einmünden, über die der Sinkleitung zu reinigendes Abwasser, Frischwasser oder eine Mischung hiervon zugeführt wird. Die Sinkleitung mündet vorzugsweise im Bereich des Zulaufverteilers im Reaktor.

Aufgrund der vorgenannten Eigenschaften und Vorteile des erfindungsgemäßen Reaktors ist dieser insbesondere als UASB-Reaktor oder als EGSB-Reaktor geeignet.

Gemäß einer alternativen Ausführungsform füllt das wenigstens eine Ablenkmittel vorzugsweise 20 bis 70 % und besonders bevorzugt 30 bis 60 % des Reaktorquerschnitts aus.

Bei dieser Ausführungsform ist das wenigstens eine Ablenkmittel vorzugsweise ringförmig ausgestaltet, da sich dadurch eine Ablenkung des dem Reaktor zugeführten Abwassers in eine kreisförmige Strömung besonders einfach und zuverlässig erreichen lässt.

Um eine stabile Kreislaufführung des dem Reaktor zugeführten Wassers zu erreichen, hat es sich bei dieser Ausführungsform zudem als vorteilhaft erwiesen, in dem Zulaufverteiler wenigstens zwei Ablenkmittel, besonders bevorzugt zwei bis fünf Ablenkmittel und ganz besonders bevorzugt zwei oder drei Ablenkmittel, vorzusehen. So ist es möglich, die einzelnen Ablenkmittel gleichmäßig über dem Querschnitt des Reaktors zu verteilen, so dass in dem Zulaufverteiler, bezogen auf den Reaktorquerschnitt, mehrere ablenkmittelfreie Bereiche vorhanden sind und die Querschnittsfläche der einzelnen Ablenkmittel vergleichsweise gering ist. Die konkrete Anzahl der Ablenkmittel hängt dabei insbesondere von der Größe des Reaktorquerschnitts ab.

Unter ringförmig wird im Sinne der vorliegenden Erfindung nicht nur ein Kreisring verstanden, sondern allgemein ein durch einen in seinem Radialschnitt in Bezug auf den Gesamtquerschnitt des Ringes dünnen Ringkörper gebildeten Ring, wobei die Grundfläche des Ringkörpers jede beliebige geometrische Form, beispielsweise eine kreisrunde, rechteckige, ovale, elliptische oder polygonale Form, annehmen kann.

Bei dieser Ausführungsform kann der Ringkörper des wenigstens einen ringförmigen Ablenkmittels innen hohl und nach unten offen sein, wobei die Austrittsöffnung der dem Ablenkmittel zugeordneten Zulaufleitung vorzugsweise in den inneren Hohlraum des Ringkörpers und besonders bevorzugt tangential zu einem gedachten, konzentrisch um die Reaktorlängsachse verlaufenden Kreis orientiert ist, so dass das aus der Austrittsöffnung der Zulaufleitung austretende Wasser auf die innere Oberfläche des Ringkörpers trifft und dadurch entsprechend umgelenkt wird. Dabei wird eine kreisförmige Umlenkung des dem Reaktor zugeführten Wassers insbesondere dann zuverlässig erreicht, wenn das ringförmige Ablenkmittel senkrecht zu der Reaktorlängsachse und konzentrisch um die Reaktorlängsachse herum angeordnet ist.

Dabei kann der Ringkörper des wenigstens einen ringförmigen Ablenkmittels im Radialschnitt umgekehrt v-förmig, umgekehrt u-förmig, rechteckig, trapezförmig oder polygonal ausgestaltet sein. Alternativ dazu kann der Ringkörper des wenigstens einen ringförmigen Ablenkmittels im Radialschnitt auch kreisförmig ausgestaltet sein, wobei der untere Teil des kreisförmigen Radialschnitts offen ist und an den beiden Öffnungsenden im Radialschnitt jeweils eine von dem Ringkörper weg weisende Schräge angebracht ist, mithin der Ringkörper des wenigstens einen ringförmigen Ablenkmittels einen im Wesentlichen schlüssellochartigen Radialschnitt aufweist. Eine andere Alternative ist es, den Ringkörper des wenigstens einen ringförmigen Ablenkmittels im Radialschnitt rautenförmig auszugestalten, wobei der untere Teil des rautenförmigen Radialschnitts offen ist und an einem der beiden Öffnungsenden oder an beiden Öffnungsenden eine Schräge angebracht ist, welche vorzugsweise von dem Ringkörper nach außen führend angeordnet ist.

Besonders gute Ergebnisse werden insbesondere erhalten, wenn das wenigstens eine ringförmige Ablenkmittel eine runde, eine ovale, eine elliptische oder eine polygonale Grundfläche aufweist.

Ferner ist es bei dieser Ausführungsform möglich, zwei identische oder im Wesentlichen gleiche ringförmige Ablenkmittel übereinanderliegend anzuordnen. Beispielsweise können zwei ringförmige Ablenkmittel mit jeweils einem umgekehrt v-förmigen Radialschnitt und einer kreisförmigen Grundfläche übereinander liegend angeordnet werden, so dass sich für diesen Aufbau ein Radialschnitt in Form eines "Doppeldachs" ergibt.

Alternativ zu dem wenigstens einen vorgenannten ringförmigen Ablenkmittel oder zusätzlich zu dem wenigstens einen vorgenannten ringförmigen Ablenkmittel kann der erfindungsgemäße Reaktor wenigstens ein innen hohles, haubenförmig ausgestaltetes Ablenkmittel enthalten, wobei das obere Teil der Haube geschlossen und das untere Teil der Haube offen ist. Vorzugsweise ist auch dieses Ablenkmittel senkrecht zur Reaktorlängsachse und konzentrisch um die Reaktorlängsachse herum angeordnet. Es ist auch möglich, dass das wenigstens eine haubenförmige Ablenkmittel die Form eines an der Grundfläche offenen, aber an den anderen Flächen geschlossenen Hohlkegels, Hohlkegelstumpfes, Hohlhalbkugel oder Hohlpyramidenstumpfes aufweist. Gute Ergebnisse werden insbesondere erhalten, wenn die Grundfläche des Hohlkegels oder des Hohlkegelstumpfes rund, oval oder elliptisch bzw. die Grundfläche des Hohlpy ramidenstumpfes polygonal, rechteckig oder quadratisch ist.

Alternativ zu einem ringförmigen und/oder haubenförmigen Ablenkmittel oder zusätzlich zu einem ringförmigen Ablenkmittel und/oder haubenförmigen Ablenkmittel kann der erfindungsgemäße Reaktor wenigstens ein Ablenkmittel aufweisen, welches die Form eines an der Grundfläche und an der Deckfläche offenen, aber an der Mantelfläche geschlossenen Hohlkegelstumpfes oder Hohlpyramidenstumpfes aufweist. Auch bei dieser Ausgestaltung des Ablenkmittels ist dieses vorzugsweise senkrecht zu der Reaktorlängsachse und konzentrisch um die Reaktorlängsachse herum angeordnet.

Die Anzahl und die Form der Ablenkmittel in dem Reaktor hängen unter anderem von dem Querschnitt des Reaktors und von der Menge des dem Reaktor pro Zeiteinheit zugeführten Abwassers ab. Während bei kleineren Reaktoren bereits ein Ablenkmittel ausreichend sein kann, beträgt die Anzahl der Ablenkmittel bei mittleren und größeren Reaktoren vorzugsweise zwischen zwei und fünf und besonders bevorzugt zwei oder drei.

Vorzugsweise sind die oberen Begrenzungen aller in dem Reaktor enthaltenen Ablenkmittel im Wesentlichen jeweils auf derselben Höhe des Reaktors angeordnet.

Ein weiterer Gegenstand der vorliegenden Erfindung ist ein Verfahren zur anaeroben Reinigung von Abwasser, wobei einem Reaktor umfassend einen Reaktorbehälter, wenigstens einen am oberen Reaktorbehälter angeordneten Überlauf zum Abführen von gereinigtem Wasser, wenigstens einen Abscheider, wenigstens eine im unteren Bereich des Reaktorbehälters angeordnete Zulaufleitung mit jeweils mindestens einer Austrittsöffnung zur Zuführung von zu reinigendem Abwasser in den Reaktor sowie wenigstens einen Zulaufverteiler zur Vermischung des dem Reaktor zugeführten Abwassers mit dem in dem Reaktor befindlichen Medium, wobei der Zulaufverteiler wenigstens ein Ablenkmittel umfasst, von dem 1 bis 80% des Reaktorquerschnitts ausgefüllt werden, zu reinigendes Abwasser zugeführt wird, wobei die Austrittsöffnung der wenigstens einen Zulaufleitung derart angeordnet ist, das wenigstens eine Ablenkmittel derart ausgestaltet ist und die Geschwindigkeit des zugeführten Abwassers derart eingestellt wird, dass das aus der Austrittsöffnung der wenigstens einen Zulaufleitung austretende Abwasser in dem Zulaufverteiler in eine, vom Reaktorquerschnitt aus gesehen, kreisförmige Strömung umgelenkt wird.

Besonders gute Ergebnisse werden insbesondere erhalten, wenn das erfindungsgemäße Verfahren in einem zuvor beschriebenen Reaktor durchgeführt wird.

Nachfolgend wird die vorliegende Erfindung rein beispielhaft anhand vorteilhafter Ausführungsformen und unter Bezugnahme auf die beigefügten Zeichnungen beschrieben.

Dabei zeigen:
- Fig. 1: eine schematische Längsschnittsansicht eines Reaktors zur anaeroben Reinigung von Abwasser gemäß eines ersten Aus- führungsbeispiels der vorliegenden Erfindung,
- Fig. 2: eine schematische Draufsicht auf den oberen Teil eines Dop- pelkonus des unteren Reaktorbehalterteils eines Reaktors zur anaeroben Reinigung von Abwasser gemäß eines zweiten Aus- führungsbeispiels der vorliegenden Erfindung,
- Fig. 3: einen Längsschnitt eines Ablenkmittels gemäß der in den Fig. 1 und 2 dargestellten Ausführungsformen und
- Fig. 4: eine schematische Draufsicht auf den oberen Teil des Doppel- konus des unteren Reaktorbehälterteils eines Reaktors zur anaeroben Reinigung von Abwasser gemäß eines dritten Aus- führungsbeispiels der vorliegenden Erfindung,
- Fig. 5: eine perspektivische Ansicht eines in dem erfindungsgemäßen Reaktor enthaltenden Zulaufverteilers gemäß einem weiteren Ausführungsbeispiel der vorliegenden Erfindung,
- Fig. 6: einen schematischen Radialschnitt eines haubenförmig, kon- zentrisch um eine Sinkleitung angeordneten Ablenkmittels sowie
- Fig. 7: einen schematischen Radialschnitt eines ringförmigen, kon- zentrisch um eine Sinkleitung angeordneten Ablenkmittels.

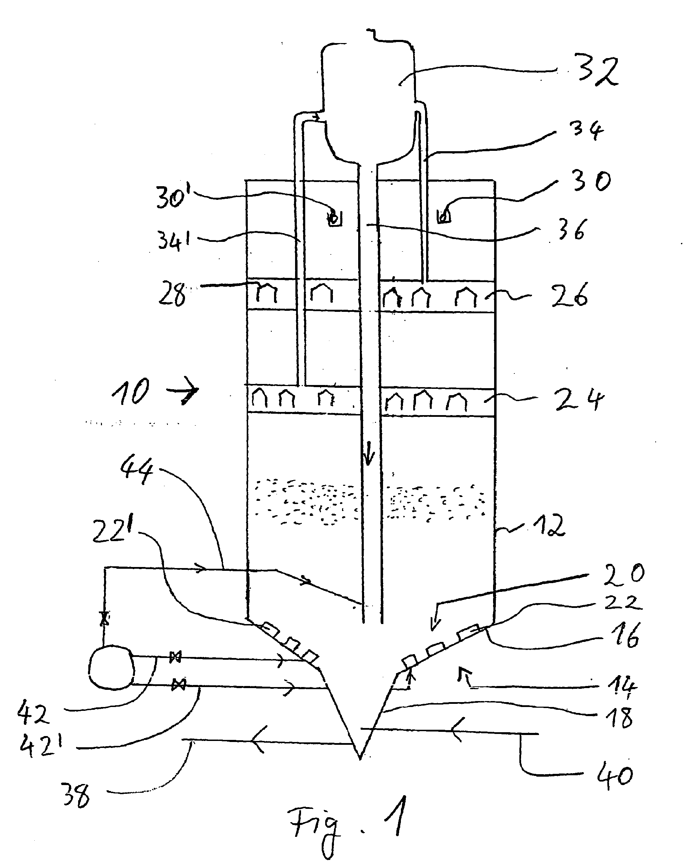

Der in der Fig. 1 schematisch im Längsschnitt dargestellte Bioreaktor 10 umfasst einen Reaktorbehälter 12, der in seinem mittleren und oberen Teil zylindrisch ausgestaltet ist und sich in seinem unteren Teil 14 nach unten doppelkonisch verjüngt, wobei der obere Teil 16 des Doppelkonus, bezogen auf die Horizontalebene, eine geringere Neigung bzw. einen kleineren Winkel als der untere Teil 18 des Doppelkonus aufweist. An dem oberen Teil 16 des Doppelkonus befindet sich ein Zulaufverteiler 20, der aus einer Vielzahl von an der Innenwand des Reaktorbehälters 12 befestigten Ablenkmitteln 22, 22' besteht, welche sich von der Reaktorwand aus in einem gewissen Winkel erstrecken und darunter befindliche, in der Wand des Reaktorbehälters 12 vorgesehene Schlitze (nicht dargestellt) überdecken. Der Zulaufverteiler 20 kann grundsätzlich auch an dem unteren Teil 18 des Doppelkonus angeordnet sein oder beispielsweise über Halterungen von der Reaktorbehälterwand 12 entfernt befestigt sein.

In dem mittleren und oberen Reaktorbehälter 12 befinden sich zwei Abscheider 24, 26, welche jeweils mehrere Gashauben 28 aufweisen. In der Praxis besteht jeder der Abscheider 24, 26 aus mehreren Lagen an Gashauben 28; in der vorliegenden Fig. 1 ist jedoch aus Einfachheitsgründen pro Abscheider 24, 26 jeweils nur eine Lage Gashauben 28 dargestellt. Alternativ dazu kann der Reaktor anstelle von zwei verschiedenen Abscheidern 24, 26 auch nur einen Abscheider 24, 26 umfassen. Oberhalb des oberen Abscheiders 26 befinden sich Überläufe 30, 30', über welche das gereinigte Wasser aus dem Reaktor 10 abgezogen wird.

Auf dem Reaktor 10 ist eine Gastrenneinrichtung 32 angeordnet, die mit den beiden Abscheidern 24, 26 über die Leitungen 34, 34' verbunden ist. Zudem führt von dem Boden der Gastrenneinrichtung 32 eine Sinkleitung 36 in den unteren Teil des Reaktors 10.

Des weiteren befindet sich im unteren Teil des Reaktors 10, nämlich im unteren Teil 18 des Doppelkonus, eine Abfuhrleitung 38 sowie eine Zufuhrleitung 40, wobei über die Abfuhrleitung 38 Feststoffe bzw. eine Suspension aus Feststoff und Flüssigkeit aus dem Reaktor 10 abgezogen werden können und über die Zufuhrleitung 40 Flüssigkeit zum Spülen des unteren Reaktorbehälterteils 14 eingeführt werden kann. Schließlich sind in dem Reaktor 10 mehrere Zulaufleitungen 42, 42' vorgesehen, die zu den einzelnen Ablenkmitteln 22, 22' führen, von denen der Übersicht halber in der Fig. 1 nur einige dargestellt sind. Zudem ist in dem Reaktor 10 eine Zulaufleitung 44 vorgesehen, welche in das untere Ende der Sinkleitung 36 mündet.

Beim Betrieb des Reaktors wird über die Zulaufleitungen 42, 42' zu reinigendes Abwasser durch die in der Wand des Reaktorbehälters 12 im oberen Teil 16 des Doppelkonus befindlichen Schlitze (nicht dargestellt) in den Reaktorbehälter 12 eingeführt und durch die Ablenkmittel 22, 22' so abgelenkt, dass das zugeführte Abwasser zu einer, vom Reaktorquerschnitt aus gesehen, kreisförmigen Strömung umgelenkt wird. Dadurch kommt es zu einer innigen Vermischung zwischen dem zugeführten Abwasser und dem in dem Reaktor 10 befindlichen Medium, welches aus bereits teilweise gereinigtem Abwasser, Mikroorganismenpellets, welche in der Fig. 1 durch kleine Punkte angedeutet sind, und kleinen Gasbläschen besteht. Das eingeführte Abwasser strömt von dem Zulaufverteiler 20 in dem Reaktorbehälter 12 langsam aufwärts, bis es in die mikroorganismenhaltige Schlammpellets enthaltende Fermentationszone gelangt. Die in den Pellets enthaltenen Mikroorganismen zersetzen die in dem Abwasser enthaltenen organischen Verunreinigungen hauptsächlich zu Methan- und Kohlendioxidgas. Durch die erzeugten Gase entstehen Gasbläschen, von denen sich die größeren von den Pellets ablösen und in Form von Gasblasen durch das Medium perlen, wohingegen kleine Gasbläschen an den Schlammpellets haften bleiben. Diejenigen Pellets, an denen kleine Gasbläschen anhaften und welche daher ein geringeres spezifisches Gewicht als die anderen Pellets und das Wasser aufweisen, steigen in dem Reaktorbehälter 12 auf, bis sie den unteren Abscheider 24 erreichen.

Die freien Gasbläschen fangen sich in den Gashauben 28 und bilden unter dem First der Gashauben 28 ein Gaspolster. Direkt unterhalb des Gaspolsters bildet sich eine Flotationsschicht bestehend aus Mikroorganismenpellets mit daran anhaftenden kleinen Gasbläschen. Das in den Gashauben 28 gesammelte Gas sowie Pellets und Wasser aus der Flotationsschicht werden beispielsweise über eine in der Stirnseite der Gashauben 28 vorhandene Öffnung (nicht dargestellt) aus den Gashauben 28 abgeführt, gegebenenfalls über eine Mischkammer (nicht dargestellt) miteinander vermischt und über die Leitung 34' in die Gastrenneinrichtung 32 geführt.

Das Wasser, die aufsteigenden Mikroorganismenpellets und die Gasblasen, die nicht bereits in dem unteren Abscheider 24 abgetrennt wurden, steigen in dem Reaktorbehälter 12 weiter nach oben bis zu dem oberen Abscheider 26. Aufgrund der Abnahme des hydrostatischen Drucks zwischen dem unteren Abscheider 24 und dem oberen Abscheider 26 lösen sich die letzten kleinen Gasbläschen von den in den oberen Abscheider 26 gelangten Mikroorganismenpellets ab, so dass das spezifische Gewicht der Pellets wieder zunimmt und die Pellets nach unten sinken. Die restlichen Gasblasen werden in den Gashauben 28 des oberen Abscheiders 26 aufgefangen und wiederum an den Stirnseiten der einzelnen Gashauben 28 in eine Gassammelleitung überführt, von der das Gas über die Leitung 34 in den Gasseparator 32 geführt wird. Das nunmehr gereinigte Wasser steigt von dem oberen Abscheider 26 weiter nach oben, bis es über die Überläufe 30, 30' aus dem Reaktor 10 abgezogen und durch eine Wasserabfuhrleitung (nicht dargestellt) abgeleitet wird.

In dem Gasseparator 32 trennt sich das Gas von dem restlichen Wasser und den Mikroorganismenpellets, wobei die Suspension aus Pellets und dem Abwasser über die Sinkleitung 36 in den Reaktorbehälter 12 rezirkuliert wird. Dabei mündet die Austrittsöffnung der Sinkleitung 36 auf der Höhe der Ablenkmittel 22, 22', wo die rückgeführte Suspension aus Pellets und Abwasser mit dem dem Reaktor 10 über die Zulaufleitungen 42, 42' zugeführten Abwasser vermischt und in eine kreisförmige Strömung versetzt wird, wonach der Kreislauf von neuem beginnt.

Über die Zulaufleitung 44 kann der Sinkleitung 36 bei Bedarf kontinuierlich oder diskontinuierlich zu reinigendes Abwasser oder Frischwasser zugeführt werden, um die durch die Sinkleitung 36 in den Reaktor 10 zurückgeführte Suspension zu verdünnen und so eine Verstopfung der Sinkleitung 36 zu verhindern.

Je nach dem Ursprung des dem Reaktor 10 über die Zulaufleitungen 42, 42' zugeführten Abwassers enthält das Abwasser mehr oder weniger Feststoffe. Abwasser aus der Papierindustrie beispielsweise enthält signifikante Konzentrationen an festen Füllmaterialien und Kalk. Nachdem das feststoffhaltige Abwasser über die Zulaufleitungen 42, 42' zu den Schlitzen und nachfolgend zu den Ablenkmitteln 22, 22' geführt wurde, wird dieses über die Ablenkmittel 22, 22' in eine, bezogen auf den Querschnitt des Reaktors 10, kreisförmige Strömung überführt und steigt, nachdem dies die Ablenkmittel 22, 22' verlassen hat, nach oben in den zylinderförmigen Reaktorbehälterteil. Der Anteil der in dem Abwasser enthaltenen Feststoffe, der ein Mindestmaß an spezifischer Dichte übersteigt, sinkt bereits nach dem Verlassen der Ablenkmittel 22, 22' in den sich nach unten verjüngenden Doppelkonus ab und sammelt sich in der unteren Spitze des unteren Teils 18 des Doppelkonus. Ferner kristallisiert ein Teil des in dem Abwasser enthaltenden Kalks, nachdem das Abwasser in die Schlammbettzone aufgestiegen ist, an den Schlammpellets aus, welche insoweit als Kristallisationszentren wirken. Dadurch übersteigt ein Teil der Schlammpellets eine kritische spezifische Dichte und sinkt infolge dessen aus dem Schlammbett ab und sammelt sich ebenfalls an der unteren Spitze des unteren Teils 18 des Doppelkonus. Aufgrund der Geometrie und Anordnung der Ablenkmittel 22, 22' wird sichergestellt, dass sich die schweren von oben nach unten sinkenden Pellets nicht auf den Ablenkmitteln 22, 22' ablagern, sondern von der Außenoberfläche der Ablenkmittel 22, 22' abrutschen und sich ebenfalls in der unteren Spitze des unteren Teils 18 des Doppelkonus ansammeln. Über die Abfuhrleitung 38 kann das sich an der unteren Spitze des Reaktors 10 sammelnde Sediment je nach Bedarf kontinuierlich oder chargenweise aus dem Reaktor 10 abgezogen werden.

Zudem kann über die Zufuhrleitung 40 ebenfalls nach Bedarf kontinuierlich oder chargenweise Wasser in den unteren Teil 18 des Doppelkonus eingeführt werden, um etwaige an den Wandungen des Doppelkonus anhaftende Sedimente aufzuwirbeln, infolge dessen diese Sedimente ebenfalls über die Abfuhrleitungen 38 aus dem Reaktor 10 abgezogen werden können. Bei dem dem Reaktor 10 über die Zufuhrleitung 40 zugeführten Wasser kann es sich um zu reinigendes Abwasser, rezirkuliertes Abwasser aus dem Reaktor, Frischwasser oder eine Mischung hiervon handeln.

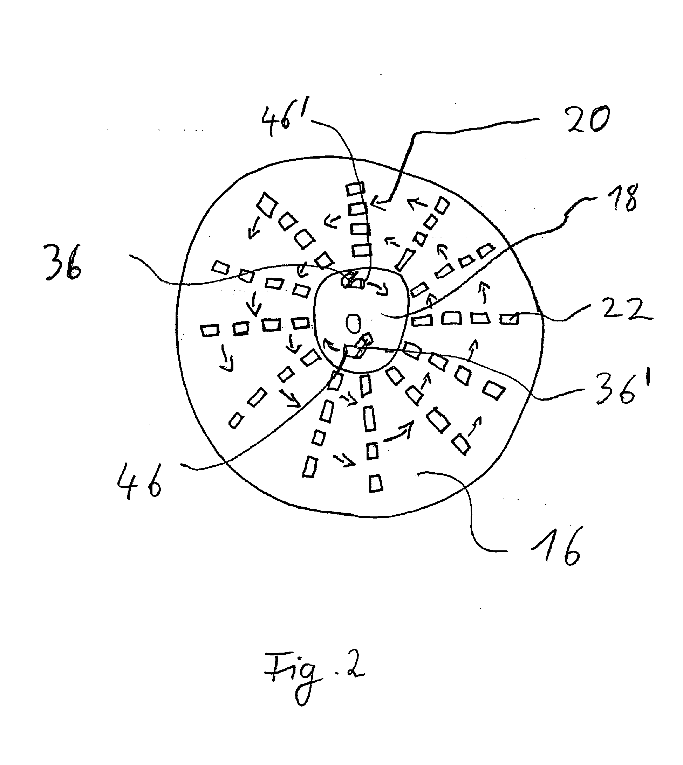

In der Fig. 2 ist der obere Teil 16 des Doppelkonus des unteren Reaktorbehälterteils 14 eines Reaktors zur anaeroben Reinigung von Abwasser gemäß eines zweiten Ausführungsbeispiels der vorliegenden Erfindung in schematischer Draufsicht dargestellt. Der in der Fig. 2 dargestellte Reaktor entspricht dem in der Fig. 1 dargestellten ausgenommen, dass der in der Fig. 2 gezeigte zwei Sinkleitungen (36, 36') umfasst.

Der Zulaufverteiler 20 umfasst insgesamt 36 rechteckige Ablenkmittel 22, von denen jeweils 4 zu einer in radialer Richtung angeordneten Reihe von versetzt zueinander, durch die Reaktorbehälterwand getrennten Ablenkmitteln 22 zusammengefasst sind, so dass der Zulaufverteiler 12 Reihen zu je vier Ablenkmitteln 22 aufweist. In dem Bereich der Ablenkmittel 22 münden die beiden Sinkleitungen 36, 36' in den Reaktor 10, wobei die Austrittsöffnungen 46, 46' der beiden Sinkleitungen 36, 36' jeweils entgegengesetzt tangential um einen konzentrisch um die Reaktorlängsachse herum gedachten Kreis angeordnet sind.

Bei der in der Fig. 2 dargestellten Ausführungsform sind demnach die einzelnen Schlitze und Ablenkmittel, von der Reaktorlängsachse aus radial nach außen gesehen, nicht flächenproportional verteilt. Alternativ zu der in der Fig. 2 dargestellten Ausführungsform können die Schlitze und Ablenkmittel auch, von der Reaktorlängsachse aus radial nach außen gesehen, flächenproportional verteilt sein. Dies kann vorzugsweise dadurch realisiert werden, dass mit zunehmendem radialen Abstand von der Reaktorlängsachse aus gesehen pro konzentrisch um die Reaktorlängsachse gedachter Kreisfläche mehr Schlitze und Ablenkmittel angeordnet werden.

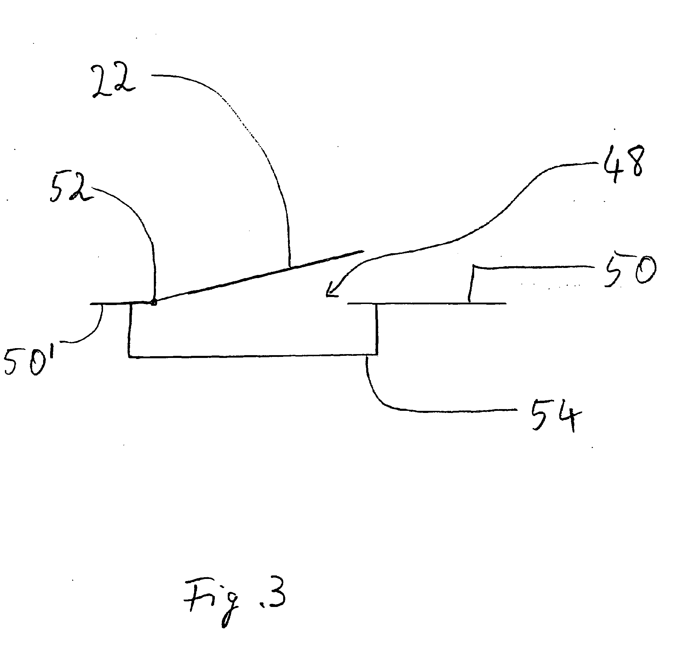

Wie aus der Fig. 3, welche einen Längsschnitt eines Ablenkmittels 24 gemäß der in den Fig. 1 und 2 dargestellten Ausführungsformen darstellt, hervorgeht befindet sich in der Reaktorwand 50, 50' des oberen Teils 16 des Doppelkonus ein rechteckiger Schlitz 48, der allseitig von der Reaktorbehälterwand 50, 50' umschlossen ist. An einer Seite des Schlitzes 48 ist ein ebenfalls rechteckig ausgebildetes Ablenkmittel 22 über ein Scharnier 52 drehbar befestigt und so justiert, dass es sich in einem bestimmten Winkel von der Reaktorbehälterwand 50, 50' aus erstreckt und den Schlitz 48 vollständig überdeckt. Unterhalb des Schlitzes 48 befindet sich eine von der nicht dargestellten Zulaufleitung radial zum Reaktorboden 50, 50' verlaufende und im Querschnitt rechteckig ausgestaltete Sammelleitung 54, über die beim Betrieb des Reaktors Wasser durch den Schlitz 48 in das Reaktorinnere eingeführt wird und dieses beim Passieren des Ablenkmittels 22 in eine, vom Reaktorquerschnitt aus gesehen, kreisförmige Strömung umgelenkt wird.

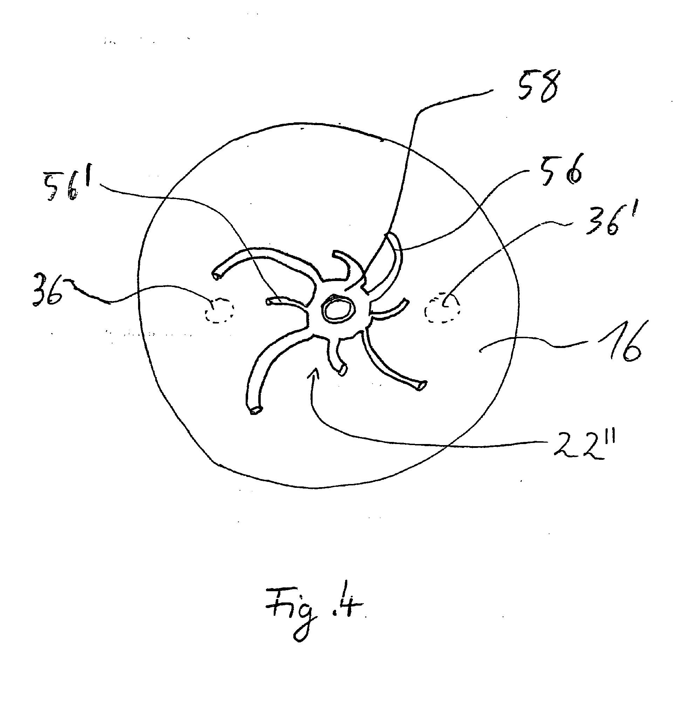

Bei der in der Fig. 4 dargestellten Ausführungsform ist das in dem oberen Teil 16 des Doppelkonus des Reaktorbehälters 12 vorgesehene Ablenkmittel 22" als planare mehrarmige Spirale ausgebildet, welche konzentrisch um die Reaktorlängsachse herum angeordnet ist. Das als planare Spirale ausgebildete Ablenkmittel 22" umfasst acht Spiralarme 56, 56', von denen vier Spiralarme 56 eine andere Länge als die restlichen vier Spiralarme 56' aufweisen. Alle Spiralarme 56, 56' weisen einen kreisförmigen Querschnitt auf und sind im Wesentlichen halbelliptisch gekrümmt ausgestaltet, wobei die Krümmungen aller Spiralarme 56, 56' in dieselbe Richtung verlaufen. Im Zentrum des spiralförmigen Ablenkmittels befindet sich eine Verteileinrichtung 58, die mit einer nicht dargestellten Zulaufleitung für Abwasser verbunden ist, wobei über die Verteileinrichtung 58 das dem Ablenkmittel 22" über diese Zulaufleitung zugeführte Abwasser auf die einzelnen Spiralarme 56, 56' verteilt wird, über deren Austrittsöffnungen das Abwasser in das Reaktorinnere eintritt und in eine, von dem Reaktorquerschnitt aus gesehen, kreisförmige Strömung umgelenkt wird. Auch der Reaktor gemäß dieser Ausführungsform umfasst zwei Sinkleitungen 36, 36'.

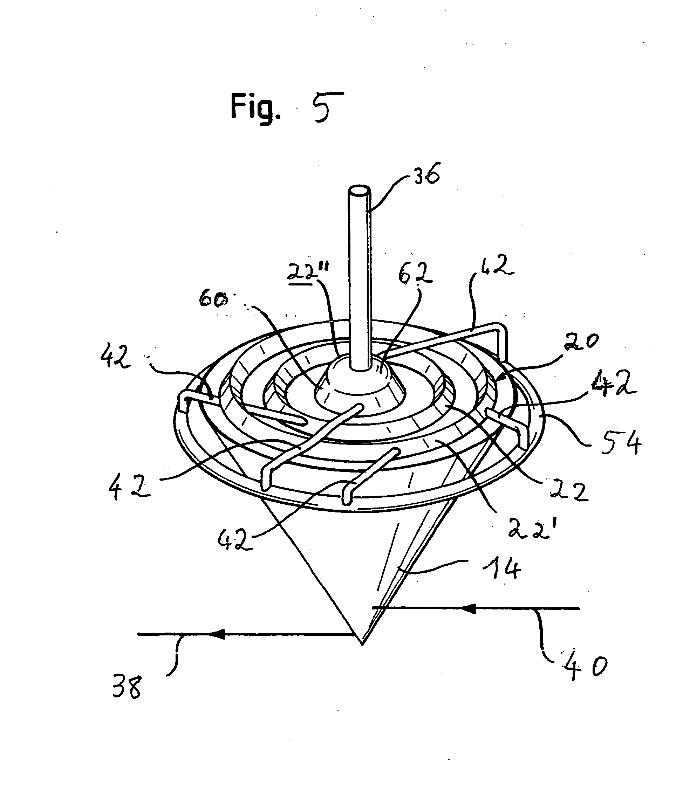

In der Fig. 5 ist ein in dem erfindungsgemäßen Reaktor 10 einsetzbarer Zulaufverteiler 20 gemäß einem weiteren Ausführungsbeispiel dargestellt. Der Zulaufverteiler 20 umfasst zwei ringförmige Ablenkmittel 22, 22', welche konzentrisch um die Reaktorlängsachse angeordnet sind. Die Ringkörper beider Ablenkmittel 22, 22' weisen einen umgekehrt v-förmigen Radialschnitt auf, wobei die Ringkörper innen hohl und nach unten offen sind. Zudem umfasst der Zulaufverteiler 20 ein haubenförmiges, konzentrisch um die Reaktorlängsachse angeordnetes Ablenkmittel 22", welches in seinem unteren Teil einen Hohlkegelstumpfabschnitt 60 und in seinem oberen Teil einen hohlkugelförmigen Abschnitt 62 umfasst. Auch das haubenförmige Ablenkmittel 22" ist innen hohl und nach unten, d.h. an der Grundfläche des Hohlkegelstumpfes 60, offen.

Der Zulaufverteiler 20 befindet sich an dem Übergang zwischen dem zylinderförmig ausgestalteten Reaktorbehälter 12 (nicht dargestellt) und dem sich nach unten verjüngenden trichterförmigen Reaktorbehälterteil 14.

Über die Zulaufleitungen 42 wird jeweils zu reinigendes Abwasser in den Hohlraum der einzelnen Ablenkmittel 22, 22', 22" eingeführt. Sämtliche Zulaufleitungen 42 entspringen einer zentralen Zulaufsammelleitung 54, welche kreisringförmig ausgestaltet ist und konzentrisch um den Zulaufverteiler 20 herum angeordnet ist.

Wie zudem aus der Fig. 5 ersichtlich, führt die konzentrisch um die Reaktorlängsachse angeordnete, von der Gastrenneinrichtung 32 kommende Sinkleitung 36 in das haubenförmige Ablenkmittel 22", wobei die Austrittsöffnung der Sinkleitung 36, knapp unterhalb der oberen Begrenzung des Ablenkmittels 22" mündet. Wie bereits dargelegt, wird über die Sinkleitung 36 während des Reaktorbetriebs eine Suspension aus Abwasser und Mikroorganismenpellets in den Bereich des Zulaufverteilers 20 zurückgeführt. Um eine Verstopfung der Sinkleitung 36 beispielsweise infolge einer hohen Konzentration von Pellets in der Suspension oder infolge eines Druckabfalls in der Sinkleitung 36 zu verhindern, kann eine weitere Zulaufleitung (nicht dargestellt) oberhalb der oberen Begrenzung des haubenförmigen Ablenkmittels 22" in die Sinkleitung 36 münden, um an der Mündungsstelle in der Sinkleitung 36 eine turbulente Strömung zu erzeugen, durch welche etwaige Verstopfungen oder Pelletagglomerate aufgrund von Scherkräften beseitigt werden.

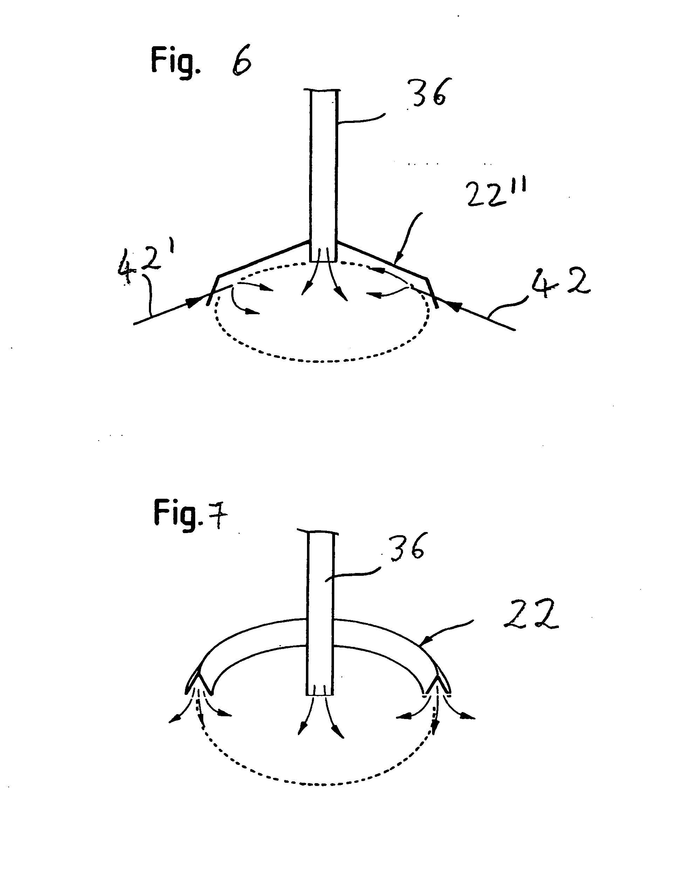

In der Fig. 6 ist der den unteren Teil der Sinkleitung 36 und das haubenförmige Ablenkmittel 22" umfassende Teil eines Zulaufverteilers 20 im schematischen Radialschnitt dargestellt, der dem in der Fig. 5 dargestellten Zulaufverteiler 20 entspricht ausgenommen, dass das haubenförmige Ablenkmittel 22" in dessen oberen Teil anstelle eines hohlkugelförmigen Abschnitts einen hohlkegelförmigen Abschnitt aufweist. Beim Betrieb des Reaktors 10 wird über die Zulaufleitungen 42, 42' zu reinigendes Abwasser in den Hohlraum des haubenförmigen Ablenkmittels 22" eingeführt.

Ferner gelangt über die Sinkleitung 36 eine Suspension an rezirkuliertem Abwasser und Mikroorganismenpellets in den Hohlraum des haubenförmigen Ablenkmittels 22". Die Strömungswege an den Austrittsöffnungen der Zulaufleitungen 42, 42' und der Sinkleitung 36 sind in der Fig. 6 durch Pfeile angedeutet. Durch die Strömungsverhältnisse entsteht in dem Hohlraum des haubenförmigen Ablenkmittels 22" eine Mischzone, welche in der Fig. 6 durch den gepunkteten Kreis angedeutet ist, in der aus der Sinkleitung 36 austretende Suspension aus Abwasser und Mikroorganismenpellets mit dem über die Zulaufleitungen 42, 42' zugeführten, zu reinigendem Abwasser innig miteinander vermischt wird.

In der Fig. 7 ist eine alternative Ausführungsform zu dem Ausführungsbeispiel der Fig. 6 dargestellt. Bei dem in der Fig. 7 gezeigten Ausführungsbeispiel ist ein ringförmiges Ablenkmittel 22 mit einem umgekehrt v-förmigen Radialschnitt konzentrisch um die Sinkleitung 36 herum angeordnet. Die Austrittsöffnung der Sinkleitung 36 befindet sich in etwa auf Höhe des unteren Endes des Ringkörpers. Auch in diesem Ausführungsbeispiel sind die Strömungsverhältnisse des durch die Sinkleitung 36 zugeführten Abwassers und der zurückgeführten Pellets sowie des durch das ringförmige Ablenkmittel 22 umgelenkten, zu reinigenden Abwassers durch Pfeile angedeutet. Auch bei diesem Ausführungsbeispiel entsteht eine in der Fig. 7 punktförmig angedeutete Mischzone, in der das über die Zulaufleitungen 42 (nicht dargestellt) zugeführte Abwasser sowie das über die Sinkleitung 36 rezirkulierte Abwasser sowie die Pellets innig miteinander vermischt werden.

### Beztlgszeichenliste

- 10: (Bio)reaktor
- 12: Reaktorbehälter
- 14: unterer, trichterförmiger Reaktorbehälterteil
- 16: oberer Teil des Doppelkonus
- 18: unterer Teil des Doppelkonus
- 20: Zulaufverteiler
- 22, 22', 22": Ablenkmittel
- 24: unterer Abscheider
- 26: oberer Abscheider
- 28: Gashaube
- 30, 30': Überlauf
- 32: Gastrenneinrichtung
- 34, 34': Leitung
- 36, 36': Sinkleitung
- 38: Abfuhrleitung
- 40: Zufuhrleitung zur Spülung
- 42, 42': Zulaufleitung zum Ablenkmittel
- 44: Zulaufleitung zu der Sinkleitung
- 46, 46': Austrittsöffnung der Sinkleitung
- 48: Schlitz
- 50, 50': Reaktorbehälterwand
- 52: Scharnier
- 54: Sammelleitung
- 56, 56': Spiralarm
- 58: Verteilereinrichtung
- 60: Hohlkegelstumpfabschnitt
- 62: hohlkugelförmiger Abschnitt

## Patentansprüche

1. Reaktor (10) zur anaeroben Reinigung von Abwasser umfassend einen Reaktorbehälter (12), wenigstens eine im unteren Bereich (14) des Reaktorbehälters (12) angeordnete Zulaufleitung (42, 42') zur Zuführung von zu reinigendem Abwasser in den Reaktor (10), wenigstens einen Zulaufverteiler (20) zur Vermischung des dem Reaktor (10) zugeführten Abwassers mit dem in dem Reaktor (10) befindlichen Medium, wenigstens einen am oberen Reaktorbehälter (12) angeordneten Überlauf (30, 30') zum Abführen von gereinigtem Wasser sowie wenigstens einen Abscheider (24, 26),
**dadurch gekennzeichnet, dass**
der Zulaufverteiler (20) wenigstens ein Ablenkmittel (22, 22', 22") umfasst, wobei das wenigstens eine Ablenkmittel (22, 22', 22") derart ausgestaltet ist, dass aus der wenigstens einen Zulaufleitung (42, 42') austretendes Abwasser in eine, vom Reaktorquerschnitt aus gesehen, kreisförmige, nach oben gerichtete Strömung umgelenkt wird, und wobei 1 bis 80 % des Reaktorquerschnitts von dem wenigstens einen Ablenkmittel (22, 22', 22") ausgefüllt werden.

2. Reaktor nach Anspruch 1,
**dadurch gekennzeichnet, dass**
von dem wenigstens einen Ablenkmittel (22, 22', 22") 2 bis 70 %, bevorzugt 3 bis 60 % und besonders bevorzugt 5 bis 50 % des Reaktorquerschnitts ausgefüllt werden.

3. Reaktor nach Anspruch 1 oder 2,
**dadurch gekennzeichnet, dass**
die wenigstens eine Zulaufleitung (42, 42') außerhalb des Reaktorbehälters (12) endet und in der Wand des unteren Bereichs (14) des Reaktorbehälters (12) wenigstens ein Schlitz (48) vorgesehen ist, durch den das aus der wenigstens einen Zulaufleitung (42, 42') austretende Abwasser in den unteren Bereich (14) des Reaktorbehälters (12) eintritt.

4. Reaktor nach Anspruch 3,
**dadurch gekennzeichnet, dass**
das im Inneren des Reaktors (10) im Bereich des wenigstens einen Schlitzes (48) wenigstens ein Ablenkmittel (22, 22') vorgesehen ist, welches derart ausgestaltet ist, dass das aus dem wenigstens einen Schlitz (48) in den Reaktor (10) eintretende Abwasser in eine, vom Reaktorquerschnitt aus gesehen, kreisförmige, nach oben gerichtete Strömung umgelenkt wird.

5. Reaktor nach Anspruch 4,
**dadurch gekennzeichnet, dass**
das wenigstens eine Ablenkmittel (22, 22') den wenigstens einen Schlitz (48) zumindest teilweise überdeckt.

6. Reaktor nach Anspruch 1 oder 2,
**dadurch gekennzeichnet, dass**
das wenigstens eine Ablenkmittel (22") als planare, mehrarmige Spirale ausgestaltet ist.

7. Reaktor nach Anspruch 6,
**dadurch gekennzeichnet, dass**
das wenigstens eine spiralförmige Ablenkmittel (22") wenigstens zwei, bevorzugt wenigstens vier und besonders bevorzugt vier bis acht Spiralarme (56, 56') aufweist.

8. Reaktor nach Anspruch 6 oder 7,
**dadurch gekennzeichnet, dass**
die einzelnen Spiralarme (56, 56') des wenigstens einen spiralförmigen Ablenkmittels (22") im Wesentlichen die Form einer Halbellipse oder eines Halbkreises aufweisen, wobei die Krümmung aller Spiralarme (56, 56') in dieselbe Richtung verläuft.

9. Reaktor nach einem der Ansprüche 6 bis 8,
**dadurch gekennzeichnet, dass**
das wenigstens eine spiralförmige Ablenkmittel (22") vier bis acht im Wesentlichen halbelliptisch oder halbkreisförmig ausgebildete Spiralarme (56, 56') umfasst, von denen zwei bis vier Spiralarme (56) eine andere Länge aufweisen als die übrigen Spiralarme (56').

10. Reaktor nach einem der Ansprüche 6 bis 9,
**dadurch gekennzeichnet, dass**
der Reaktor eine Zulaufleitung (42, 42') und der Zulaufverteiler (20) ein spiralförmiges Ablenkmittel (22") sowie eine Verteileinrichtung (58) aufweist, wobei die Zulaufleitung (42, 42') in dem Zentrum des einen Ablenkmittels (22") in die Verteileinrichtung (58) mündet und das durch die Zulaufleitung geführte Abwasser über den Verteiler (58) auf die einzelnen Spiralarme (56, 56') des Ablenkmittels (22") verteilt wird.

11. Reaktor nach einem der vorhergehenden Ansprüche,
**dadurch gekennzeichnet, dass**
der untere Teil des Reaktors (10) die Form eines sich nach unten verjüngenden Einfachkonus oder Doppelkonus aufweist.

12. Reaktor nach Anspruch 11,
**dadurch gekennzeichnet, dass**
am unteren Ende des Konus eine Abfuhrleitung (38) angeordnet ist.

13. Reaktor nach Anspruch 11 oder 12,
**dadurch gekennzeichnet, dass**
im unteren Bereich des Konus eine Zulaufleitung (40) zur Spülung des Reaktorbodens angeordnet ist.

14. Verfahren zur anaeroben Reinigung von Abwasser, wobei einem Reaktor (10) umfassend einen Reaktorbehälter (12), wenigstens einen am oberen Reaktorbehälter (12) angeordneten Überlauf (30, 30') zum Abführen von gereinigtem Wasser, wenigstens einen Abscheider (24, 26), wenigstens eine im unteren Bereich des Reaktorbehälters (12) angeordnete Zulaufleitung (42, 42') mit jeweils mindestens einer Austrittsöffnung zur Zuführung von zu reinigendem Abwasser in den Reaktor (10) sowie wenigstens einen Zulaufverteiler (20) zur Vermischung des dem Reaktor (10) zugeführten Abwassers mit dem in dem Reaktor (10) befindlichen Medium, wobei der Zulaufverteiler (20) wenigstens ein Ablenkmittel (22, 22', 22") umfasst, von dem 1 bis 80% des Reaktorquerschnitts ausgefüllt werden, zu reinigendes Abwasser zugeführt wird, wobei die Austrittsöffnung der wenigstens einen Zulaufleitung (42, 42') derart angeordnet ist, das wenigstens eine Ablenkmittel (22, 22', 22") derart ausgestaltet ist und die Geschwindigkeit des zugeführten Abwassers derart eingestellt wird, dass das aus der Austrittsöffnung der wenigstens einen Zulaufleitung (42, 42') austretende Abwasser in dem Zulaufverteiler (20) in eine, vom Reaktorquerschnitt aus gesehen, kreisförmige, nach oben gerichtete Strömung umgelenkt wird.

15. Verfahren nach Anspruch 14,
**dadurch gekennzeichnet, dass**
dieses in einem Reaktor (10) nach einem der Patentansprüche 2 bis 13 durchgeführt wird.

## Claims

1. A reactor (10) for the anaerobic treatment of waste water comprising a reactor tank (12), at least one inflow line (42, 42') arranged in the lower region (14) of the reactor tank (12) for the supply of waste water to be treated into the reactor (10), at least one inflow distributor (20) for the mixing of the waste water supplied to the reactor (10) with the medium located in the reactor (10), at least one overflow (30, 30') arranged at the upper region of the reactor tank (12) for the draining off of treated water as well as at least one separator (24, 26),
**characterized in that**
the inflow distributor (20) comprises at least one deflection means (22, 22', 22"), with the at least one deflection means (22, 22' 22") being designed such that waste water emerging from the at least one inflow line (42, 42') is deflected into a circular, upwardly directed flow, viewed from the reactor cross-section, and with 1 to 80% of the reactor cross-section being filled by the at least one deflection means (22, 22', 22").

2. A reactor in accordance with claim 1, **characterized in that** 2 to 70%, preferably 3 to 6% and particularly preferably 5 to 50% of the reactor cross-section is filled by the at least one deflection means (22, 22', 22").

3. A reactor in accordance with claim 1 or claim 2, **characterized in that** the at least one inflow line (42, 42') ends outside the reactor tank (12) and at least one slot (48) is provided in the wall of the lower region (14) of the reactor tank (12), with the waste water emerging from the at least one inflow line (42, 42') entering into the lower region (14) of the reactor tank (12) through said at least one slot.

4. A reactor in accordance with claim 3, **characterized in that** at least one deflection means (22, 22') is provided in the interior of the reactor (10) in the region of the at least one slot (48) and is made such that the waste water entering into the reactor (10) from the at least one slot (48) is deflected into a circular, upwardly directed flow, viewed from the reactor cross-section.

5. A reactor in accordance with claim 4, **characterized in that** the at least one deflection means (22, 22') covers the at least one slot (48) at least partly.

6. A reactor in accordance with claim 1 or claim 2, **characterized in that** the at least one deflection means (22") is made as a planar, multi-arm spiral.

7. A reactor in accordance with claim 6, **characterized in that** the at least one spiral deflection means (22") has at least two, preferably at least four and particularly preferably four to eight spiral arms (56, 56').

8. A reactor in accordance with claim 6 or claim 7, **characterized in that** the individual spiral arms (56, 56') of the at least one spiral deflection means (22") substantially have the shape of a semi-ellipse or of a semi-circle, with the curvature of all the spiral arms (56, 56') extending in the same direction.

9. A reactor in accordance with any one of the claims 6 to 8, **characterized in that** the at least one spiral deflection means (22") comprises four to eight substantially semi-elliptical or semi-circular spiral arms (56, 56') of which two to four spiral arms (56) have a different length than the other spiral arms (56').

10. A reactor in accordance with any one of the claims 6 to 9, **characterized in that** the reactor has an inflow line (42, 42') and the inflow distributor (20) has a spiral deflection means (22") and a distribution device (58), with the inflow line (42, 42') opening into the distribution device (58) at the center of the one deflection means (22") and the waste water guided by the inflow line being distributed via the distributor (58) to the individual spiral arms (56, 56') of the deflection means (22").

11. A reactor in accordance with any one of the preceding claims, **characterized in that** the lower part of the reactor (10) has the shape of a downwardly converging simple cone or double cone..

12. A reactor in accordance with claim 11, **characterized in that** a drainage line (38) is arranged at the lower end of the cone.

13. A reactor in accordance with claim 11 or claim 12, **characterized in that** an inflow line (40) is arranged in the lower region of the cone for the flushing of the reactor base.

14. A method for the anaerobic treatment of waste water, wherein waste water to be treated is supplied to a reactor (10) comprising a reactor tank (12), at least one overflow (30, 30') arranged at the upper region of the reactor tank (12) for the draining off of treated water, at least one separator (24, 26), at least one inflow line (42, 42') arranged in the lower region of the reactor tank (12) and having in each case at least one outlet opening for the supply of waste water to be treated into the reactor (10) as well as at least one inflow distributor (20) for the mixing of the waste water supplied to the reactor (10) with the medium located in the reactor, (10), wherein the inflow distributor comprises at least one deflection means (22, 22', 22") by which 1 to 80% of the reactor cross-section is filled, wherein the outlet opening of the at least one inflow line (42, 42') is arranged such that the at least one deflection means (22, 22', 22") is designed and the speed of the supplied waste water is set such that the waste water emerging from the outlet opening of the at least one inflow line (42, 42') is deflected in the inflow distributor (20) into a circular, upwardly directed flow, viewed from the reactor cross-section.

15. A method in accordance with claim 14, **characterized in that** it is carried out in a reactor (10) in accordance with any one of the claims 2 to 13.

## Revendications

1. Réacteur (10) pour l'épuration anaérobie d'eaux usées, comprenant un récipient de réacteur (12), au moins une conduite d'amenée (42, 42') agencée dans la région inférieure (14) du récipient de réacteur (12) pour l'admission des eaux usées à épurer dans le réacteur (10), au moins un répartiteur d'amenée (20) pour mélanger les eaux usées amenées au réacteur (10) avec le milieu qui se trouve dans le réacteur (10), au moins un déversement (30, 30') agencé en partie haute du récipient de réacteur (12) pour évacuer l'eau épurée, ainsi qu'au moins un séparateur (24, 26),
**caractérisé en ce que**
le répartiteur d'amenée (20) comprend au moins un moyen de déviation (22, 22', 22"), tel que ledit au moins un moyen de déviation (22, 22', 22") est conçu de telle manière que les eaux usées sortant de ladite au moins une conduite d'amenée (42, 42') sont déviées pour donner un écoulement dirigé vers le haut et en forme circulaire, vu depuis la section du réacteur, et de sorte que 1 à 80 % de la section du réacteur sont remplies par ledit au moins un moyen de déviation (22, 22', 22").

2. Réacteur selon la revendication 1,
**caractérisé en ce que** 2 à 70 %, de préférence 3 à 60 % et de manière particulièrement préférée 5 à 50 % de la section de réacteur sont remplies par ledit au moins un moyen de déviation (22, 22', 22").

3. Réacteur selon la revendication 1 ou 2,
**caractérisé en ce que** ladite au moins une conduite d'amenée (42, 42') se termine à l'extérieur du récipient de réacteur (12), et dans la paroi de la région inférieure (14) du récipient de réacteur (12) il est prévu au moins une fente (48) à travers laquelle les eaux usées qui sortent de ladite au moins une conduite d'amenée (42, 42') pénètrent dans la région inférieure (14) du récipient de réacteur (12).

4. Réacteur selon la revendication 3,
**caractérisé en ce qu'**il est prévu à l'intérieur du réacteur (10) dans la région de ladite au moins une fente (48) au moins un moyen de déviation (22, 22') qui est conçu de telle manière que les eaux usées qui pénètrent hors de ladite au moins une fente (48) dans le réacteur (10) sont déviées pour donner un écoulement dirigé vers le haut et de forme circulaire, vu depuis la section du réacteur.

5. Réacteur selon la revendication 4,
**caractérisé en ce que** ledit au moins un moyen de déviation (22, 22') recouvre au moins partiellement ladite au moins une fente (48).

6. Réacteur selon la revendication 1 ou 2,
**caractérisé en ce que** ledit au moins un moyen de déviation (22") est conçu sous la forme d'une spirale plane à plusieurs bras.

7. Réacteur selon la revendication 6,
**caractérisé en ce que** ledit au moins un moyen de déviation (22") en forme de spirale comprend au moins deux, de préférence au moins quatre, et de façon particulièrement préférée de quatre à huit bras de spirale (56, 56').

8. Réacteur selon la revendication 6 ou 7,
**caractérisé en ce que** les bras de spirale (56, 56') individuels dudit au moins un moyen de déviation (22") en forme de spirale présentent essentiellement la forme d'une demi-ellipse ou d'un demi-cercle, la courbure de tous les bras de spirale (56, 56') s'étendant dans la même direction.

9. Réacteur selon l'une des revendications 6 à 8,
**caractérisé en ce que** ledit au moins un moyen de déviation (22") en forme de spirale comprend quatre à huit bras de spirale réalisés sensiblement en forme de demi-ellipse ou de demi-cercle, parmi lesquels deux à quatre bras de spirale (56) présentent une autre longueur que les bras de spirale restants (56').

10. Réacteur selon l'une des revendications 6 à 9,
**caractérisé en ce que** le réacteur comprend une conduite d'amenée (42, 42') et le répartiteur d'amenée (20) comprend un moyen de déviation (22") en forme de spirale ainsi qu'un système de répartition (58), tel que la conduite d'amenée (42, 42') débouche dans le système de répartition (58) au centre du moyen de déviation (22") concerné, et les eaux usées menées à travers la conduite d'amenée sont réparties via le répartiteur (58) vers les bras de spirale (56, 56') individuels du moyen de déviation (22").

11. Réacteur selon l'une des revendications précédentes,
**caractérisé en ce que** la partie inférieure du réacteur (10) présente la forme d'un cône simple ou d'un double cône qui va en se rétrécissant vers le bas.

12. Réacteur selon la revendication 11,
**caractérisé en ce qu'**une conduite d'évacuation (38) est agencée à l'extrémité inférieure du cône.

13. Réacteur selon la revendication 11 ou 12,
**caractérisé en ce qu'**une conduite d'amenée (40) destinée au rinçage du fond de réacteur est agencée dans la région inférieure du cône.

14. Procédé pour l'épuration anaérobie d'eaux usées, dans lequel des eaux usées à épurer sont amenées à un réacteur (10) qui comprend un récipient de réacteur (12), au moins un déversement (30, 30') agencé en partie supérieure du récipient de réacteur (12) pour l'évacuation d'eau épurée, au moins un séparateur (24, 26), au moins une conduite d'amenée (42, 42') agencée dans la région inférieure du récipient de réacteur (12), avec respectivement au moins une ouverture de sortie pour l'admission des eaux usées à épurer dans le réacteur (10), ainsi qu'au moins un répartiteur d'amenée (20) pour le mélange des eaux usées admises dans le réacteur (10) avec le milieu qui se trouve dans le réacteur (10), dans lequel le répartiteur d'amenée (20) comprend au moins un moyen de déviation (22, 22', 22") avec lequel 1 à 80 % de la section du réacteur sont remplis, ladite ouverture de sortie de ladite au moins une conduite d'amenée (42, 42') est agencée de telle manière, ledit au moins un moyen de déviation (22, 22', 22") est conçu de telle manière, et la vitesse des eaux usées admises est réglée de telle manière que les eaux usées qui sortent de l'ouverture de sortie de ladite au moins une conduite d'amenée (42, 42') sont déviées pour donner un écoulement dirigé vers le haut et de forme circulaire vu depuis la section du réacteur, dans le répartiteur d'amenée (20).

15. Procédé selon la revendication 14,
**caractérisé en ce que** celui-ci est mis en oeuvre dans un réacteur (10) selon l'une des revendications 2 à 13.
